# EUROPEAN PATENT APPLICATION

(11) **EP 3 292 870 A1**
(43) Date of publication of application: **14.03.2018**
(21) Application number: 16187926.7
(22) Date of filing: 08.09.2016
(51) Int. Cl.: A61K 38/39, C07K 14/78, C07K 14/82

(54) **FIBRONECTIN FOR USE IN THE TREATMENT OF LEUKEMIA**

(71) Applicant: Chemotherapeutisches Forschungsinstitut Georg-Speyer-Haus, 60596 Frankfurt (DE)
(72) Inventor: KRAUSE, Daniela S., 60598 Frankfurt am Main (DE); MEISTER, Melanie, 64283 Darmstadt (DE); KUMAR, Rahul, 60596 Frankfurt am Main (DE)
(74) Representative: Kuttenkeuler, David

(57) **Abstract**

The present invention pertains to fibronectin in the treatment of imatinib resistant leukemia. The invention relates to the use of recombinant or isolated fibronectin as adjuvant therapy during leukemia treatment, either as single ingredient medicament or in a combination therapy, preferably with Abl1 inhibitors such as imatinib or nilotinib.

## Description

### FIELD OF THE INVENTION

The present invention pertains to fibronectin in the treatment of imatinib resistant leukemia. The invention relates to the use of recombinant or isolated fibronectin as adjuvant therapy during leukemia treatment, either as single ingredient medicament or in a combination therapy, preferably with ABL inhibitors such as imatinib or nilotinib.

### DESCRIPTION

Chronic Myelogenous Leukemia (CML) is a hematological disorder which constitutes about 15% of adult leukemia, characterized by the malignant expansion of the myeloid lineage. The genetic hallmark of CML is a reciprocal translocation between chromosomes 9 and 22 resulting in the so- called Philadelphia (Ph) chromosome. The molecular consequence of this inter-chromosomal exchange is the creation of the chimeric gene BCR-ABL1, encoding a tyrosine kinase polypeptide in which the tyrosine kinase domain is constitutively activated. The expression of this fusion protein has been shown to be necessary and sufficient for the transformed phenotype of CML cells. In addition to CML, deregulated ABL kinase activity resulting from the Ph chromosomal translocation is also detected in up to 20% of adult lymphoblastic leukemia (ALL) patients.

Hematological cancers have a high frequency amongst the general population and pose a major health care problem. Significant progress has been made in the treatment of leukemia. The advent of the BCR-ABL1-targeting tyrosine kinase inhibitor (TKI) imatinib mesylate (IM) (marketed as Gleevec® or Glivec®) in 2001 increased the 5-year survival rate of patients to 90%. However, leukemic stem cells (LSC) are not eradicated (Corbin AS, Agarwal A, Loriaux M, Cortes J, Deininger MW, Druker BJ. Human chronic myeloid leukemia stem cells are insensitive to imatinib despite inhibition of BCR-ABL activity. J Clin Invest. 2011;121(1):396.) and 90% of patients relapse with mutations in BCR-ABL1 that lead to resistance towards IM (Shah NP, Nicoll JM, Nagar B, Gorre ME, Paquette RL, Kuriyan J, et al. Multiple BCR-ABL kinase domain mutations confer polyclonal resistance to the tyrosine kinase inhibitor imatinib (STI571) in chronic phase and blast crisis chronic myeloid leukemia. Cancer Cell. 2002;2(2):117-25). Strikingly, most mutations leading to resistance towards IM occur in the Abl1 kinase domain, such as the point mutations of threonine 315 into isoleucine (BCR-ABL1T315I). The BCR-ABL1T315I mutation accounts for approximately 15% of all mutations found in patients during second line therapy, and for approximately 30% of mutations in patients on third line therapy (Soverini S, Branford S, Nicolini FE, Talpaz M, Deininger MW, Martinelli G, et al. Implications of BCR-ABL1 kinase domain-mediated resistance in chronic myeloid leukemia. Leuk Res. 2014;38(1):10-20.). Interestingly, patients with the BCR-ABL1^{T315I} mutation have a rapid clinical course and poor prognosis, while for instance another mutation, the BCR-ABL1M351T mutation, is associated with slower clinical progression (Branford S, Rudzki Z, Walsh S, Parkinson I, Grigg A, Szer J, et al. Detection of BCR-ABL mutations in patients with CML treated with imatinib is virtually always accompanied by clinical resistance, and mutations in the ATP phosphate-binding loop (P-loop) are associated with a poor prognosis. Blood. 2003;102:276-83.). Second and third line TKIs such as nilotinib and dasatinib do not target the BCR-ABL1^{T315I} mutation and ponatinib, which is effective against the BCR-ABL1^{T315I} mutation, (and nilotinib and dasatinib) are frequently associated with severe adverse events (Moslehi JJ, Deininger M. Tyrosine Kinase Inhibitor-Associated Cardiovascular Toxicity in Chronic Myeloid Leukemia. J Clin Oncol. 2015;33(35):4210-8.), rendering the therapy for BCR-ABL1^{T315I}+ CML or B-cell acute lymphoblastic leukemia (B-ALL) extremely difficult and frequently unsuccessful. A novel strategy for targeting BCR-ABL1^{T315I}+ CML is needed.

The BMM or bone marrow niche represents the very complex arrangement of various cell types, all of which form the bone marrow niche for normal hematopoietic stem cells (HSC), the normal counterparts to LSC. Other factors determining the (patho-) physiology of the BMM are the oxygen tension, pH, mechanical forces, the extracellular matrix and cytokines. These various constituents affect the number, location, proliferation, self-renewal, and differentiation of hematopoietic stem and progenitor cells (HSPC) and likely similarly influence LSC. LSC interact with the BMM via specific pathways and are protected by the BMM to prevent chemotherapy- and TKI-induced apoptosis. As evidence of the BMM being a targetable entity in leukemia, it was previously demonstrated that modulation of the BMM by parathyroid hormone, the most potent regulator of bone turnover, led to a reduction of LSC in CML, but not AML, suggesting differential effects of the niche on myeloid leukemia (Krause DS, Fulzele K, Catic A, Sun CC, Dombkowski D, Hurley M, et al. Differential regulation of myeloid leukemia by the bone marrow microenvironment. Nat Med. 2013;19(11):1513-7).

It was therefore an object of the present invention to provide therapeutic options to provide novel treatments of leukemia. A further object of the invention was to overcome imatinib resistance during leukemia treatment.

The above problem is solved in a first aspect by fibronectin, or a functional variant thereof, as well as salts, solvates and/or derivatives thereof, for use in the treatment or prevention of cancer.

The term "fibronectin", or "FN", as used in the present invention pertains to a protein expressed by a fibronectin gene as well as nucleic acid sequences encoding for the protein. The term shall comprise the various isoforms of fibronectin that occur through alternative splicing. Preferred are fibronectin proteins that are derived from the human fibronectin (*fn1*) gene locus. The *fn1* gene has the Genbank accession number 2335, and is furthermore annotated in other databases, such as in Ensembl:ENSG00000115414 HPRD:00626; MIM:135600; Vega:OTTHUMG00000133054. The expression of its various splice forms is well known in the art and can be derived for example from Schwarzbauer et al 2011 ("Fibronectins, their fibrillogenesis, and in vivo functions."-, Schwarzbauer JE1, DeSimone DW; Cold Spring Harb Perspect Biol. 2011 Jul 1;3(7). pii: a005041. doi: 10.1101/cshperspect.a005041). The fibronectin protein may be provided as a monomer or a dimer. Fibronectin is known to occur in a soluble form (formerly known as cold-insoluble globulin or CIg), which is a soluble FN dimer in which two FN monomers are covalently connected via disulfide bridges. This form occurs in the plasma and is a preferred form for use according to the present invention. However, FN protein is also present in higher order structures in an insoluble fibrillary form in the extracellular matrix.

In context of the invention the term fibronectin may both refer to the protein or the nucleic acid encoding such a protein. Therefore the invention in some embodiments also relates to a fibronectin encoding nucleic acid for use in the treatment or prevention of cancer. For example, the fibronectin encoding nucleic acid may occur in the form of an expression vector comprising a fibronectin encoding nucleic acid operably linked to a promoter sequence.

A fibronectin in context of the invention is in some embodiments preferably in the form of the isolated recombinant and/or purified protein in a composition, which means that the protein is in a non-naturally occurring composition prepared for the therapeutic administration to a mammal. Such a composition may be serum or plasma preparation, or a blood preparation with an increased concentration of soluble fibronectin. In some other embodiments the fibronectin composition of the invention is not a blood transfusion, plasma or serum preparation, but a composition wherein fibronectin is the only blood derived component. In some embodiments the fibronectin composition is a cryoprecipitate.

A proteinaceous fibronectin for use according to the present invention is either a recombinantly produced protein, or a protein purified from a blood or plasma sample from a donor. The donor may be selected from a healthy donor or from an autologous blood or plasma sample. The FN protein for use according to the invention is preferably a full length human fibronectin, comprising not more than 20 mutations, wherein a mutation is selected from an amino acid substitution, deletion, insertion or addition, compared to the sequence of any isoform of human fibronectin as known in the art.

The term "cancer" shall refer to any proliferative cancerous disorders, and preferably includes solid and liquid cancers. Most preferably a cancer of the invention is leukemia. The term "leukemia" refers broadly to progressive, malignant diseases of the blood-forming organs and is generally characterized by a distorted proliferation and development of leukocytes and their precursors in the blood and bone marrow. Leukemia is generally clinically classified on the basis of the duration and character of the disease-acute or chronic; the type of cell involved; myeloid (myelogenous), lymphoid, or monocytic; and the increase or non-increase in the number of abnormal cells in the blood-leukemic or aleukemic (subleukemic). Accordingly, the present invention includes fibronectin for use in treating leukemia, including treating acute myeloid leukemia, chronic lymphocytic leukemia, acute promyelocytic leukemia, adult T-cell leukemia, aleukemic leukemia, a leukocythemic leukemia, basophilic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocyte leukemia, micro myeloblastic leukemia, monocytic leukemia, myeloblastic leukemia, myelocytic leukemia, myeloid granulocytic leukemia, myelomonocytic leukemia, Naegeli leukemia, plasma cell leukemia, multiple myeloma, plasmacytic leukemia, promyelocytic leukemia, Rieder cell leukemia, Schilling's leukemia, stem cell leukemia, subleukemic leukemia, and undifferentiated cell leukemia.

Most preferably treated in context with the present invention is a Bcr-Abl1 positive leukemia, such as a Chronic Myelogenous Leukemia (CML) or Acute Lymphocytic Leukemia (ALL). Also it is preferable in context of the invention that the leukemia is an imatinib-resistant leukemia, preferably a Bcr-Abl1 mutant positive, preferably imatinib resistant, leukemia. A Bcr-Abl1 mutant in context of the invention is preferably selected from the group consisting of M351T, F359V, H396P, I432T, F486S, M244V, L248V, G250E, Y253H, Y253F, E255K, K263E, L273M, T315I, F317L, and N331S; and preferably is the Bcr-Abl^{T315I} mutant.

The treatment of leukemia according to the invention may furthermore comprise chemotherapy, radiation therapy or stem cell transplantation approaches. As used herein, the term "bone marrow transplantation" or "stem cell transplantation" or "hematopoietic stem cell transplantation" used herein should be considered as interchangeable, referring to the transplantation of stem cells in some form to a recipient. The terms shall include both allogenic as well as autologous stem cell transplantations. The stem cells do not necessarily have to be derived from bone marrow, but could also be derived from other sources such as umbilical cord blood.

Therefore, in some embodiments the fibronectin may be used in context of a stem cell or bone marrow transplantation for a leukemia therapy according to the invention, wherein the to be transplanted stem cells are contacted with fibronectin before transplantation, or wherein the fibronectin is administered concomitantly with the stem cells to the patient.

Preferably, the treatment according to the invention comprises the administration of a therapeutically effective amount of fibronectin, preferably to the bone marrow of a subject suffering from the cancer. The administration of fibronectin may be local, however, the administration is preferably systemic, for example by intravenous injection.

A systemic administration in context of the present invention refers to oral, rectal, and parenteral (i.e., intramuscular, intravenous, and subcutaneous) routes for administration. Generally, it will be found that when the compounds of the invention are administered orally a larger quantity of the active agent is required to produce the same effect as a smaller quantity given parenterally. In accordance with good clinical practice, it is preferred to administer the compounds at a concentration level that will produce effective therapeutic effect without causing any harmful or untoward side effects.

The "therapeutically effective dose" of the compounds of the invention in a composition for purposes herein is determined by such considerations as are known in the art. The dose must be effective to achieve improvement including but not limited to an improved course of disease, more rapid recovery, and improvement of symptoms, elimination of symptoms and other indicators as are selected as appropriate measures by those skilled in the art. The compounds of the invention can be administered in a single dose or in multiple doses.

In general, the active dose of compound for humans is in the range of from 1 ng/kg to about 20-100 mg/kg body weight per day, preferably about 0.01 mg/kg to about 2-10 mg/kg body weight per day, in a regimen of one dose per day or twice or three or more times per day for a single dose or multiple dose regimen.

In some embodiments it will be preferred that fibronectin is used as an adjuvant during a cancer treatment. In these embodiments the treatment according to the invention comprises the concomitant or sequential use of fibronectin and at least one other anti-cancer agent. Alternatively, the treatment comprises the use of a combination of fibronectin or a functional variant thereof, as well as salts, solvates and/or derivatives thereof, and at least one other anti-cancer agent.

An "Anti-cancer agent" in accordance with the invention shall refer to a composition (e.g. compound, drug, antagonist, inhibitor, modulator) having antineoplastic properties or the ability to inhibit the growth or proliferation of cells. In embodiments, an anticancer agent is a chemotherapeutic. In embodiments, an anti-cancer agent is an agent approved by the EMA, or FDA, or similar regulatory agency of a country other than the European Union, for treating cancer. Examples of anti-cancer agents include, but are not limited to, MEK (e.g. MEK1, MEK2, or MEK1 and MEK2) inhibitors (e.g. XL518, CI-1040, PD035901, selumetinib/ AZD6244, GSK1120212/ trametinib, GDC-0973, ARRY-162, ARRY-300, AZD8330, PD0325901, U0126, PD98059, TAK-733, PD318088, AS703026, BAY 869766), alkylating agents (e.g., cyclophosphamide, ifosfamide, chlorambucil, busulfan, melphalan, mechlorethamine, uramustine, thiotepa, nitrosoureas, nitrogen mustards (e.g., mechloroethamine, cyclophosphamide, chlorambucil, melphalan), ethylenimine and methylmelamines (e.g., hexamethlymelamine, thiotepa), alkyl sulfonates (e.g., busulfan), nitrosoureas (e.g., carmustine, lomustine, semustine, streptozocin), triazenes (decarbazine)), anti-metabolites (e.g., 5- azathioprine, leucovorin, capecitabine, fludarabine, gemcitabine, pemetrexed, raltitrexed, folic acid analog (e.g., methotrexate), or pyrimidine analogs (e.g., fluorouracil, floxouridine, Cytarabine), purine analogs (e.g., mercaptopurine, thioguanine, pentostatin), etc.), plant alkaloids (e.g., vincristine, vinblastine, vinorelbine, vindesine, podophyllotoxin, paclitaxel, docetaxel, etc.), topoisomerase inhibitors (e.g., irinotecan, topotecan, amsacrine, etoposide (VP 16), etoposide phosphate, teniposide, etc.), antitumor antibiotics (e.g., doxorubicin, adriamycin, daunorubicin, epirubicin, actinomycin, bleomycin, mitomycin, mitoxantrone, plicamycin, etc.), platinum-based compounds (e.g. cisplatin, oxaloplatin, carboplatin), anthracenedione (e.g., mitoxantrone), substituted urea (e.g., hydroxyurea), methyl hydrazine derivative (e.g., procarbazine), or adrenocortical suppressant (e.g., mitotane, aminoglutethimide), epipodophyllotoxins (e.g., etoposide).

Further examples of anti-cancer agents include, but are not limited to, antibiotics (e.g., daunorubicin, doxorubicin, bleomycin), enzymes (e.g., L-asparaginase), inhibitors of mitogen- activated protein kinase signaling (e.g. U0126, PD98059, PD184352, PD0325901, ARRY- 142886, SB239063, SP600125, BAY 43-9006, wortmannin, or LY294002), mTOR inhibitors, antibodies (e.g., rituxan), 5-aza-2'-deoxycytidine, doxorubicin, vincristine, etoposide, gemcitabine, imatinib (Gleevec.RTM.), geldanamycin, 17-N-Allylamino-17- Demethoxygeldanamycin (17-AAG), bortezomib, trastuzumab, anastrozole; angiogenesis inhibitors; antiandrogen, antiestrogen; antisense oligonucleotides; apoptosis gene modulators; apoptosis regulators; arginine deaminase; BCR/ABL1 antagonists; beta lactam derivatives; bFGF inhibitor; bicalutamide; camptothecin derivatives; casein kinase inhibitors (ICOS); clomifene analogues; cytarabine dacliximab; dexamethasone; estrogen agonists; estrogen antagonists; etanidazole; etoposide phosphate; exemestane; fadrozole; finasteride; fludarabine; fluorodaunorunicin hydrochloride; gadolinium texaphyrin; gallium nitrate; gelatinase inhibitors; gemcitabine; glutathione inhibitors; hepsulfam; immunostimulant peptides; insulin-like growth factor- 1 receptor inhibitor; interferon agonists; interferons; interleukins; letrozole; leukemia inhibiting factor; leukocyte alpha interferon; leuprolide+estrogen+progesterone; leuprorelin; matrilysin inhibitors; matrix metalloproteinase inhibitors; MIF inhibitor; mifepristone; mismatched double stranded RNA; monoclonal antibody,; mycobacterial cell wall extract; nitric oxide modulators; oxaliplatin; panomifene; pentrozole; phosphatase inhibitors; plasminogen activator inhibitor; platinum complex; platinum compounds; prednisone; proteasome inhibitors; protein A-based immune modulator; protein kinase C inhibitor; protein kinase C inhibitors, protein tyrosine phosphatase inhibitors; purine nucleoside phosphorylase inhibitors; ras farnesyl protein transferase inhibitors; ras inhibitors; ras-GAP inhibitor; ribozymes; signal transduction inhibitors; signal transduction modulators; single chain antigen-binding protein; stem cell inhibitor; stem-cell division inhibitors; stromelysin inhibitors; synthetic glycosaminoglycans; tamoxifen methiodide; telomerase inhibitors; thyroid stimulating hormone; translation inhibitors; tyrosine kinase inhibitors; urokinase receptor antagonists; steroids (e.g., dexamethasone), finasteride, aromatase inhibitors, gonadotropinreleasing hormone agonists (GnRH) such as goserelin or leuprolide, adrenocorticosteroids (e.g., prednisone), progestins (e.g.,hydroxyprogesterone caproate, megestrol acetate, medroxyprogesterone acetate), estrogens (e.g., diethlystilbestrol, ethinyl estradiol), antiestrogen (e.g., tamoxifen), androgens (e.g., testosterone propionate, fluoxymesterone), antiandrogen (e.g., flutamide), immunostimulants (e.g., Bacillus Calmette-Guerin (BCG), levamisole, interleukin-2, alpha- interferon, etc.), monoclonal antibodies (e.g., anti-CD20, anti-HER2, anti-CD52, anti-HLA-DR, and anti-VEGF monoclonal antibodies), immunotoxins (e.g., anti-CD33 monoclonal antibody-calicheamicin conjugate, anti-CD22 monoclonal antibody-pseudomonas exotoxin conjugate, etc.), radio immunotherapy (e.g., anti- CD20 monoclonal antibody conjugated to U 1ln, 90Y, or 131I, etc.), triptolide, homoharringtonine, dactinomycin, doxorubicin, epirubicin, topotecan, itraconazole, vindesine, cerivastatin, vincristine, deoxyadenosine, sertraline, pitavastatin, irinotecan, clofazimine, 5- nonyloxytryptamine, vemurafenib, dabrafenib, erlotinib, gefitinib, EGFR inhibitors, epidermal growth factor receptor (EGFR)-targeted therapy or therapeutic (e.g. gefitinib (Iressa™), erlotinib (Tarceva™), cetuximab (Erbitux™), lapatinib (Tykerb™), panitumumab (Vectibix™), vandetanib (Caprelsa™), afatinib/BIBW2992, CI-1033/canertinib, neratinib/HKI-272, CP-724714, TAK-285, AST-1306, ARRY334543, ARRY-380, AG-1478, dacomitinib/PF299804, OSI-420/desmethyl erlotinib, AZD8931, AEE788, pelitinib/EKB-569, CUDC-101, WZ8040, WZ4002, WZ3146, AG-490, XL647, PD153035, BMS-599626), sorafenib, imatinib, sunitinib, nilotinib, dasatinib, or the like.

In some preferred embodiments of the invention the least one anti-cancer agent is a kinase inhibitor, preferably a tyrosine kinase inhibitor such as an inhibitor of Platelet-derived growth factor receptor (PDGFR), c-Abl and/or Src kinase inhibitors.

Yet further preferable is that the tyrosine kinase inhibitor for use in the combinatorial treatments of the invention is imatinib or nilotinib, or derivatives and/or salts thereof, such as the methanesulfonate salt of imatinib (also known as Gleevec®). Imatinib mesylate is chemically 4-[(4-Methyl 1-piperazinyl)methyl]-N-[4- methyl-3-[[4-(3-pyridinyl)-2-pyrimidinyl]amino]-phenyl]benzamide methanesulfonate and is used to treat chronic myelogenous leukemia (CML), gastrointestinal stromal tumors (GISTs) and other cancers.

The term "nilotinib" as used herein includes nilotinib in the form of free base, a pharmaceutically acceptable salt thereof, amorphous nilotinib, crystalline nilotinib or any isomer, derivative, hydrate, solvate, or prodrug or combinations thereof. "Salts" or "pharmaceutically acceptable salt(s)", as used herein, include but are not limited to inorganic or organic salts, hydrates and solvates of nilotinib known to persons skilled in the art.

The subject to be treated with the fibronectin in accordance with the present invention is preferably a subject suffering from a leukemic disorder. Preferably such a subject is a naive patient who did not receive any treatment. Alternatively preferred is that the subject in need of the treatment of the invention is a subject that already underwent a treatment, such as a treatment with a tyrosine kinase inhibitor, e.g. imatinib. In some embodiments the subject is a refractory subject, hence, a subject who underwent a treatment with for example imatinib, and the treatment was successful but after the treatment experienced a relapse. Most preferably the subject to be treated in context of the invention is a patient who suffers from an imatinib resistant form of leukemia.

In a further aspect of the present invention here is provided a combination comprising (a) fibronectin, or a functional variant thereof, as well as salts, solvates and/or derivatives there-of, and (b) Bcr-Abl1-inhibitor, the latter being preferably selected from imatinib and nilotinib as defined herein above.

The combination of the invention is some embodiments preferably is for use in the treatment of leukemia, as described herein above.

Yet another aspect of the invention then pertains to a pharmaceutical composition comprising fibronectin, or a functional variant thereof, as well as salts, solvates and/or derivatives thereof, and a pharmaceutically acceptable carrier, stabilizer and/or excipient. The pharmaceutical composition of the invention is preferably for the above described medical uses, in particular for use in the treatment of leukemia.

Pharmaceutical compositions comprising the fibronectin compounds of the invention are administered to a subject in need thereof by any number of routes including, but not limited to, topical, oral, intravenous, intramuscular, intra-arterial, intramedullary, intrathecal, intraventricular, transdermal, subcutaneous, intraperitoneal, intranasal, enteral, topical, sublingual, or rectal means.

In accordance with one embodiment, a method of treating a subject in need of such treatment is provided. The method comprises administering a pharmaceutical composition comprising a fibronectin of the present invention to a subject in need thereof.

The pharmaceutical compositions useful for practicing the invention may be administered to deliver a dose of between 1 ng/kg/day and 100 mg/kg/day.

The formulations of the pharmaceutical compositions described herein may be prepared by any method known or hereafter developed in the art of pharmacology. In general, such preparatory methods include the step of bringing the active ingredient into association with a carrier or one or more other accessory ingredients, and then, if necessary or desirable, shaping or packaging the product into a desired single- or multi-dose unit.

It will be understood by the skilled artisan that such pharmaceutical compositions are generally suitable for administration to animals of all sorts. Subjects to which administration of the pharmaceutical compositions of the invention is contemplated include, but are not limited to, humans and other primates, mammals including commercially relevant mammals such as cattle, pigs, horses, sheep, cats, and dogs, birds including commercially relevant birds such as chickens, ducks, geese, and turkeys. The invention is also contemplated for use in contraception for nuisance animals such as rodents.

A pharmaceutical composition of the invention may be prepared, packaged, or sold in bulk, as a single unit dose, or as a plurality of single unit doses. As used herein, a "unit dose" is a discrete amount of the pharmaceutical composition comprising a predetermined amount of the active ingredient. The amount of the active ingredient is generally equal to the dosage of the active ingredient which would be administered to a subject or a convenient fraction of such a dosage such as, for example, one-half or one-third of such a dosage.

The relative amounts of the active ingredient, the pharmaceutically acceptable carrier, and any additional ingredients in a pharmaceutical composition of the invention will vary, depending upon the identity, size, and condition of the subject treated and further depending upon the route by which the composition is to be administered. By way of example, the composition may comprise between 0.1% and 100% (w/w) active ingredient.

In addition to the active ingredient, a pharmaceutical composition of the invention may further comprise one or more additional pharmaceutically active agents, preferably anti-cancer agent.

Controlled- or sustained-release formulations of a pharmaceutical composition of the invention may be made using conventional technology.

As used herein, "additional ingredients" include, but are not limited to, one or more of the following: excipients; surface active agents; dispersing agents; inert diluents; granulating and disintegrating agents; binding agents; lubricating agents; sweetening agents; flavoring agents; coloring agents; preservatives; physiologically degradable compositions such as gelatin; aqueous vehicles and solvents; oily vehicles and solvents; suspending agents; dispersing or wetting agents; emulsifying agents, demulcents; buffers; salts; thickening agents; fillers; emulsifying agents; antioxidants; antibiotics; antifungal agents; stabilizing agents; and pharmaceutically acceptable polymeric or hydrophobic materials. Other "additional ingredients" which may be included in the pharmaceutical compositions of the invention are known in the art and described, for example in Genaro, ed., 1985, Remington's Pharmaceutical Sciences, Mack Publishing Co., Easton, Pa., which is incorporated herein by reference. Preferable is that the additional agents are suitable for the formulation of proteinaceous active ingredients.

Typically, dosages of the compound of the invention which may be administered to an animal, preferably a human, range in amount from 1 µg to about 100 g per kilogram of body weight of the animal. While the precise dosage administered will vary depending upon any number of factors, including but not limited to, the type of animal and type of disease state being treated, the age of the animal and the route of administration. In one aspect, the dosage of the compound will vary from about 1 mg to about 10 g per kilogram of body weight of the animal. In another aspect, the dosage will vary from about 10 mg to about 1 g per kilogram of body weight of the animal.

The compound may be administered to an animal as frequently as several times daily, or it may be administered less frequently, such as once a day, once a week, once every two weeks, once a month, or even less frequently, such as once every several months or even once a year or less. The frequency of the dose will be readily apparent to the skilled artisan and will depend upon any number of factors, such as, but not limited to, the type of cancer being diagnosed, the type and severity of the condition or disease being treated, the type and age of the animal, etc.

Suitable preparations include injectables, either as liquid solutions or suspensions, however, solid forms suitable for solution in, suspension in, liquid prior to injection, may also be prepared. The preparation may also be emulsified, or the polypeptides encapsulated in liposomes. The active ingredients are often mixed with excipients which are pharmaceutically acceptable and compatible with the active ingredient. Suitable excipients are, for example, water saline, dextrose, glycerol, ethanol, or the like and combinations thereof. In addition, if desired, the vaccine preparation may also include minor amounts of auxiliary substances such as wetting or emulsifying agents, pH buffering agents, and/or adjuvants.

The present invention will now be further described in the following examples with reference to the accompanying figures and sequences, nevertheless, without being limited thereto. For the purposes of the present invention, all references as cited herein are incorporated by reference in their entireties. In the Figures:
- **Figure 1:**: Measurement of the shortest three-dimensional distance of a) normal (black) or BCR-ABL1+ (gray) lin- c-Kit+ Sca-1+ (LKS) or LKS CD150+ CD48-(LKS SLAM) cells, b) vehicle (black)- or imatinib (gray)-treated BCR-ABL1+ LKS cells and c) BCR-ABL1WT+ (black) versus BCR-ABL1T315I+ (gray) LKS cells labeled with the lipophilic dye DiD and injected into irradiated Col2.3kbGFP mice to the endosteum. The horizontal black line represents the mean.
- **Figure 2:**: Adhesion of sorted Mac-1+ BCR-ABL1T315I-positive cells from mice with CML to a) fibronectin or b) a murine stroma cell line.
- **Figure 3:**: Kaplan-Meier-survival curve of Balb/c recipient mice that were transplanted with BCR-ABL1WT (orange), BCR-ABL1Y253F (green), BCR-ABL1E255K (red), BCR-ABL1T315I (purple) and BCR-ABL1M351T (yellow) transduced bone marrow.
- **Figure 4:**: Southern blot from DNA isolated from mouse spleen from either control mice, BCR-ABL1T315IWT or BCR-ABL1T315I mice using a probe to GFP (a) and the quantification of clones per lane (b).
- **Figure 5:**: Immunofluorescence for fibronectin of 3T3 fibroblasts transduced with retroviruses expressing BCR-ABL1WT (left) or BCR-ABL1T315I (right).
- **Figure 6:**: Immunohistochemistry for fibronectin on bone sections of mice transplanted with empty-vector (left)-, BCR-ABL1 (middle) or BCR-ABL1T315I (right)-transduced cells taken at the time the mice were moribund.
- **Figure 7:**: White blood cell counts per µl in peripheral blood (a) and survival (b) of mice intravenously transplanted (no FN) or intrafemorally co-transplanted with BCR-ABL1 (gray colors)- or BCR-ABL1T315I (blue colors)-transduced bone marrow and vehicle or FN.
- **Figure 8:**: Kaplan-Meier survival curve of mice intravenously transplanted with BCR-ABL1 (gray colours) or BCR-ABL1T315I (blue colours)-transduced bone marrow treated with vehicle or FN on days 9, 10 and 12 after transplantation.

### EXAMPLES

### Example 1: Hematopoietic stem cells and leukemia-initiating cells occupy distinct niches in the BMM

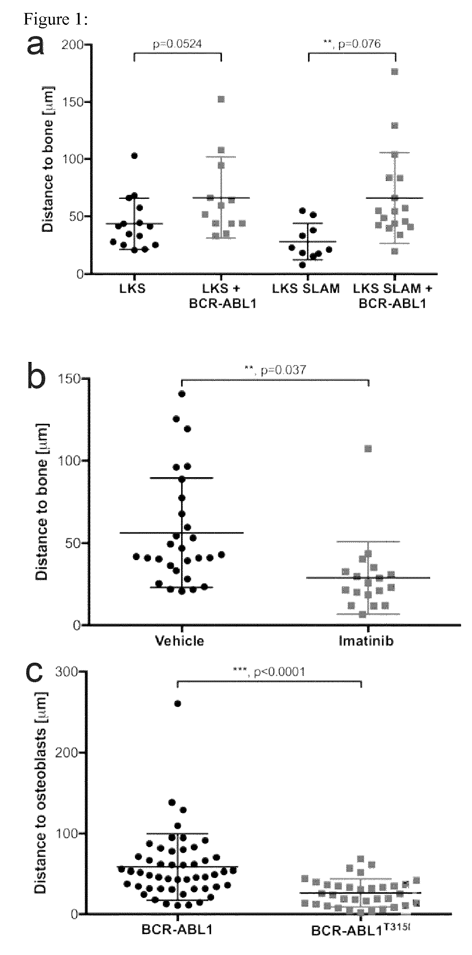

Using confocal 2-photon microscopy of the murine calvarium (skull) (15) and the murine retroviral transduction/transplantation model of CML induced by BCR-ABL1 or BCR-ABLI^{T315I} (16), the inventors could show that a) BCR-ABL1^{WT}-positive LSC home to locations further away from bone than normal HSC (Fig. 1 a), b) that imatinib-treatment reverses this phenotype and leads to closer localization of BCR- ABL1WT+ LSC to the endosteum than treatment with vehicle (Fig. 1 b) and c) that LSC positive for BCR- ABL1^{T315I} homed significantly closer to the bone than BCR-ABL1^{WT} LSC (Fig. 1 c).

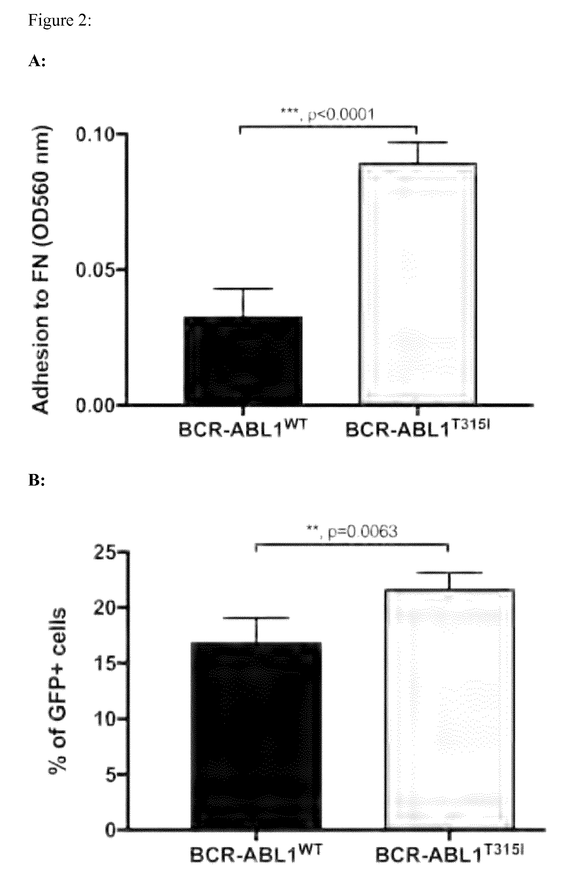

Furthermore, increased adhesion of BCR-ABL1^{T315I}+ compared to BCR-ABL1WT+ myeloid cells to fibronectin (Fig. 2 a) and stroma (Fig. 2 b) was demonstrated by an adhesion assay, respectively, suggesting that an altered interaction with the BMM may be associated with altered clinical outcome.

### Example 2: CML survival depends on the mutation status in the BCR-ABL1 kinase

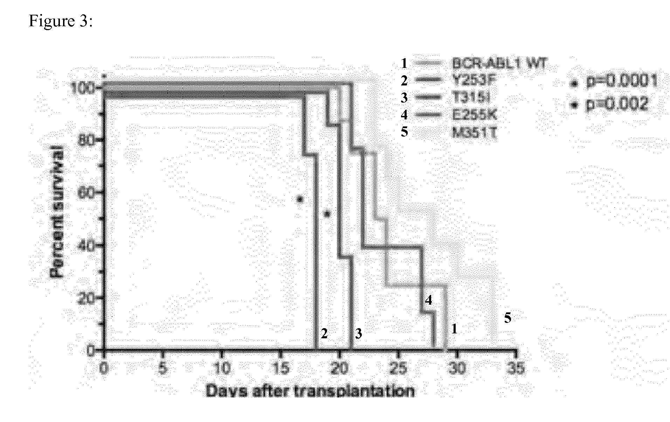

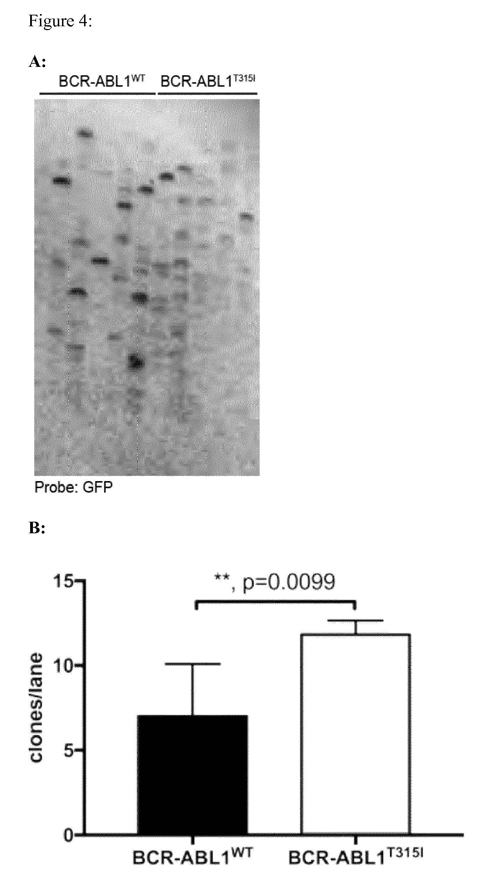

In the same murine model recipients of bone marrow transduced with BCR-ABL1^{WT} died by day 30, while recipients of BCR-ABL1^{M351T} -transduced bone marrow had prolonged and recipients of BCR-ABL1^{T315I} or BCR-ABL1^{Y253F}-transduced bone marrow had shortened survival (Fig. 3), exactly recapitulating the survival in human patients (1). Consistently, measurement of the engraftment of BCR-ABL1+ clones by Southern blotting (9, 14) revealed an increase in clonality of the BCR-ABL1^{T315I}+ compared to the BCR-ABL1^{W1}+ disease (Fig. 4a and 4h; P<0.01), suggesting that the increased engraftability likely correlates with clinical course (9, 14) and possibly localization in the niche.

### Example 3: Interaction with fibronectin influences the aggressivity of CML

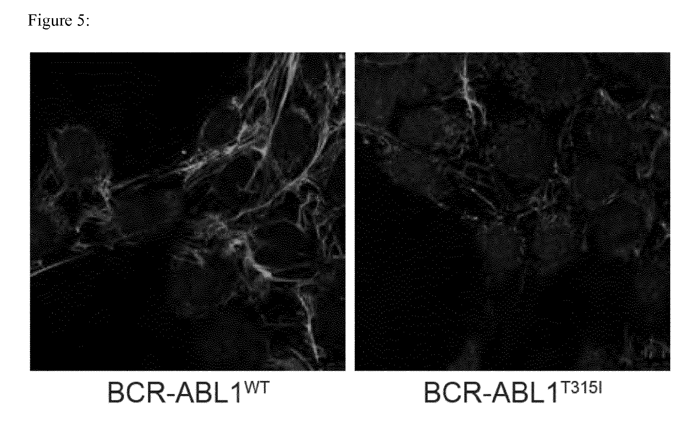

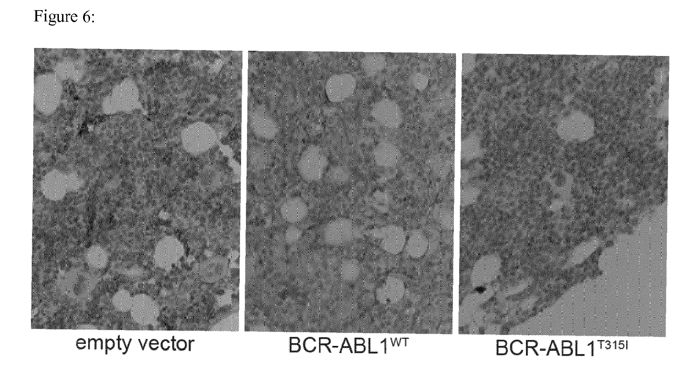

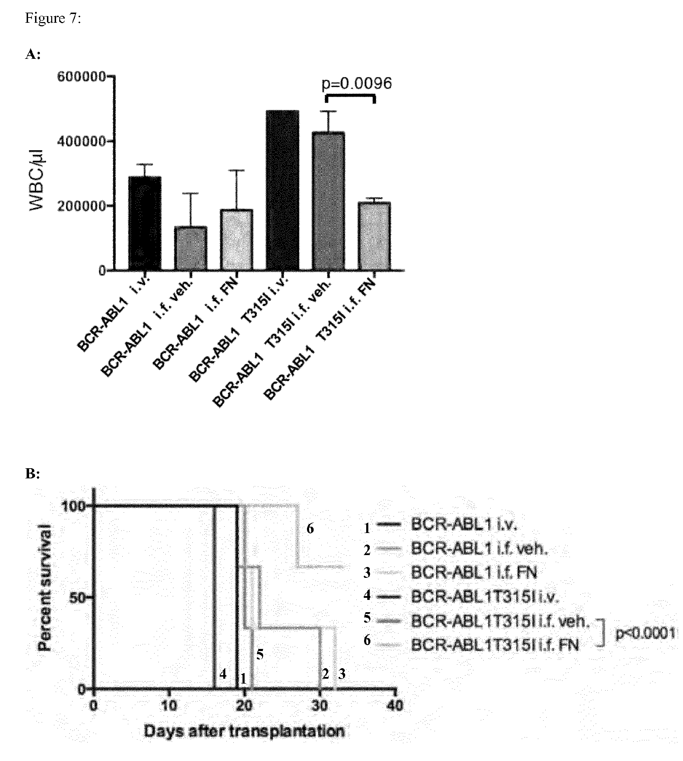

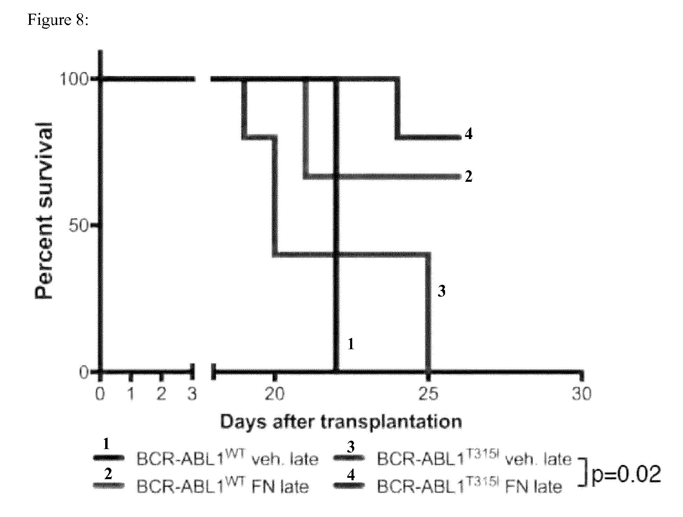

The inventors have shown by immunofluorescence (Fig. 5) and immunohistochemistry of bone sections of mice transplanted with empty vector-transduced cells, or mice with BCR-ABL1^{WT}+ or BCR-ABL1^{T315I}+ CML-like myeloproliferative neoplasia (Fig. 6), that BCR-ABL1^{T315I}+ cells deposit less fibronectin in their environment than BCR-ABL1^{WT}+ cells, which is likely also reflected by their differences in their interaction with the BMM. Importantly, intrafemoral co-injection of fibronectin and BCR-ABL1^{WT}+ or BCR-ABL1^{T315I}+ leukemia-initiating cells significantly decreased the tumor burden (Fig. 7a) and prolonged survival in recipients of BCR-ABL1^{T315I}-transduced bone marrow compared to recipients of the same bone marrow but intrafemorally co-injected with vehicle (Fig. 7b). In a more translational experiment it was further demonstrated that three intravenous injections of fibronectin on days 9, 10 and 12 post transplantation led to a significant prolongation of survival in BCR-ABL1^{T315I}+ CML compared to vehicle-treated mice with this disease (Fig. 8).

Taken together, the data suggest that substitution of (pathologically decreased) fibronectin in the BMM of mice with BCR-ABL1^{T315I}+ CML leads to reduced aggressivity and prolonged survival in this extremely aggressive form of leukemia.

Depending on the prior treatment regimen the BCR-ABL1^{T315I} mutation occurs in approximately 15-30% of all CML patients in whom mutations conveying resistance to TKIs have been found. In addition, the BCR-ABL1^{T315I} mutation can be found in blastic phase CML and in B-ALL. The presence of this mutation is frequently associated with worse outcome and treatment options are limited, especially in view of the frequent side effects of ponatinib. Novel treatments, ideally those with a different mode of attack, are urgently needed.

Intravenous injection or transfusion of fibronectin according to the herein described invention is a feasible novel form of treatment for BCR-ABL1^{T315I}+ CML and possibly B-ALL. Fibronectin is present in plasma and enriched in cryoprecipitate, a form of plasma, which was frozen, thawed to a slush stage, centrifuged and frozen. Fibronectin could, therefore, be obtained from healthy blood donors or may be produced recombinantly.

### Materials and Methods

### Antibodies and Reagents

Immunohistochemistry: Fibronectin (Abcam, ab2413, Cambridge, UK); Immunofluorescence: AlexaFluor-647 goat anti-rabbit (Molecular Probes/Thermo Fisher, Waltham, USA), fibronectin (Abcam, ab2413, Cambridge, UK).

Murine fibronectin was purchased from Abcam (ab92784, Cambridge, UK), lyophilized bovine fibronectin was purchased from Thermo Fisher (33010018, Waltham, USA, prepared as 1 mg/ml in HBSS).

### Mice

BALB/c mice were purchased from Charles River Laboratories (Sulzfeld, Germany). All animal studies were approved by the government in the German region of Hessen (Regierungsprasidium Darmstadt).

### Bone marrow transduction and transplantation

Our retroviral stock was generated and bone marrow transplantation experiments were performed as described earlier (9, 14, 16). In general, we injected between 2.25 and 2.5 x 105 transduced cells into female irradiated (750 cGy) Balb/c recipient mice. The T315I point mutation in BCR-ABL1 was introduced by site-directed mutagenesis in the open reading frame of the MSCV IRES GFP vector.

### Treatment of mice

Therapeutic application of fibronectin was performed as follows: intravenous application of 200 µg bovine fibronectin (in HBSS) on days 9, 10 and 12 post transplantation; for vehicle control treatments, 200 µl of HBSS (equal volume to fibronectin dose) was administered. For intrafemoral injections, 50 µg murine fibronectin was injected on days 0, 1 and 2 post transplantation; for vehicle control treatments, 50 µl HBSS was used.

### Fibronectin Adhesion Assay

Adhesion to fibronectin was tested as described by the manufacturer (Cell Biolabs Inc., San Diego, USA). In brief, 150.000 cells per fibronectin-coated well of a 24-well-plate were allowed to adhere for 90 min. After vigorous washing with PBS cells were stained with the supplied staining solution and after washing with water extracted with the supplied extraction solution. Wells coated with bovine serum albumin (BSA) were used as control. Adhesion was measured in a spectrophotometer at OD560.

### Immunofluorescence

Transduced 3T3 fibroblasts were allowed to adhere and to grow on round coverslips of a 15 mm diameter for at least 24 hours. Alternatively, non-adherent BaF3 or primary bone marrow cells were cytospun onto polysine-coated slides (Menzel Gläser. Braunschweig). Cells were either fixed in pure methanol for 10 min at -20°C or in 4% paraformaldehyde (PFA) (Morphisto, Franfurt am Main) for 10 min at room temperature. PFA-fixed cells were permeabilised with 0.25% Triton in PBS for 5 min. Prior to incubation with primary antibodies cells were blocked in 2% BSA in PBS for 10 min at room temperature. Primary antibodies were generally diluted 1:100 in 2% BSA/PBS and cells were incubated for 1 h at room temperature. After 2 washing steps in PBS for 5 min each, the cells were incubated for 1 h at room temperature with fluorophore-labeled secondary antibodies (diluted 1:300), including a counterstain for nuclei with 5 µg/ml DAPI (Merck, Darmstadt). Cells were washed in PBS and briefly in water and mounted with FluoroMount plus 50 µg/ml 1,4-diazabicyclo(2,2,2)octan (DABCO) (Sigma-Aldrich, Munich). Specimen were analyzed using a confocal laser scanning microscope (Leica, SP5, Wetzlar) and pictures were managed and analyzed with ImageJ.

### Histology/Immunohistochemistry

Bones were immersed in formalin for at least 24 h. Consequently, bones were decalcified with 0.5 M EDTA for 5 days with an exchange of EDTA after the first 24 h. Bones were then kept in formalin until mounting in paraffin. Bones were sectioned, de-paraffinised and either stained with hematoxylin & eosin for histopathological analysis or with an antibody directed to fibronectin (Abcam, ab2413, Cambridge UK) following standard procedures.

### Southern Blotting

DNA was extracted using phenol/chloroform and precipitated with isopropanol. The DNA was then digested with the restriction endonuclease BglII, separated by agarose gel electrophoresis and transferred to a nylon membrane. Subsequently, DNA was hybridized with a radioactively labeled probe derived from the GFP gene to detect proviral integration sites, as described (14).

### Statistical analysis

All statistical analyses were performed using GraphPad Prism software. Statistical tests such as unpaired, two-tailed Student's t-test, one- or two-way ANOVA tests were used where appropriate. In general, p-values below 0.05 were considered significant (p<0.05, *). Survival curves were analysed with Kaplan-Meier-style survival curves and log-rank (Mantel-Cox) or Gehan-Breslow-Wilcoxon tests.

References:
1. Branford S, Rudzki Z, Walsh S, Parkinson I, Grigg A, Szer J, et al. Detection of BCR-ABL mutations in patients with CML treated with imatinib is virtually always accompanied by clinical resistance, and mutations in the ATP phosphate-binding loop (P-loop) are associated with a poor prognosis. Blood. 2003;102:276-83.
2. Corbin AS, Agarwal A, Loriaux M, Cortes J, Deininger MW, Druker BJ. Human chronic myeloid leukemia stem cells are insensitive to imatinib despite inhibition of BCR-ABL activity. J Clin Invest. 2011;121(1):396.
3. Shah NP, Nicoll JM, Nagar B, Gorre ME, Paquette RL, Kuriyan J, et al. Multiple BCR-ABL kinase domain mutations confer polyclonal resistance to the tyrosine kinase inhibitor imatinib (STI571) in chronic phase and blast crisis chronic myeloid leukemia. Cancer Cell. 2002;2(2): 117-25.
4. Soverini S, Branford S, Nicolini FE, Talpaz M, Deininger MW, Martinelli G, et al. Implications of BCR-ABL1 kinase domain-mediated resistance in chronic myeloid leukemia. Leuk Res. 2014;38(1):10-20.
5. Moslehi JJ, Deininger M. Tyrosine Kinase Inhibitor-Associated Cardiovascular Toxicity in Chronic Myeloid Leukemia. J Clin Oncol. 2015;33(35):4210-8.
6. Krause DS, Scadden DT. A hostel for the hostile: the bone marrow niche in hematologic neoplasms. Haematologica. 2015;100(11):1376-87.
7. Lundell BI, Mc Carthy JB, Kovach NL, Verfaillie CM. Activation of beta1 integrins on CML progenitors reveals cooperation between beta1 integrins and CD44 in the regulation of adhesion and proliferation. Leukemia. 1997;11:822-9.
8. Tanaka R, Owaki T, Kamiya S, Matsunaga T, Shimoda K, Kodama H, et al. VLA-5-mediated adhesion to fibronectin accelerates hemin-stimulated erythroid differentiation of K562 cells through induction of VLA-4 expression. J Biol Chem. 2009;284(30):19817-25.
9. Krause DS, Lazarides K, von Andrian UH, Van Etten RA. Requirement for CD44 in homing and engraftment of BCR-ABL-expressing leukemic stem cells. Nat Med. 2006;12(10):1175-80.
10. Krause DS, Lazarides K, Lewis JB, von Andrian UH, Van Etten RA. Selectins and their ligands are required for homing and engraftment of BCR-ABL1+ leukemic stem cells in the bone marrow niche. Blood. 2014;123(9):1361-71.
11. Jin L, Hope KJ, Zhai Q, Smadja-Joffe F, Dick JE. Targeting of CD44 eradicates human acute myeloid leukemic stem cells. Nat Med. 2006;12(10):1167.
12. Ishikawa F, Yoshida S, Saito Y, Hijikata A, Kitamura H, Tanaka S, et al. Chemotherapy-resistant human AML stem cells home to and engraft within the bone-marrow endosteal region. Nat Biotechnol. 2007;25(11):1315-21.
13. Zhang B, Li M, McDonald T, Holyoake TL, Moon RT, Campana D, et al. Microenvironmental protection of CML stem and progenitor cells from tyrosine kinase inhibitors through N-cadherin and Wnt-beta-catenin signaling. Blood. 2013;121 (10):1824-38.
14. Krause DS, Fulzele K, Catic A, Sun CC, Dombkowski D, Hurley M, et al. Differential regulation of myeloid leukemias by the bone marrow microenvironment. Nat Med. 2013;19(11):1513-7.
15. Lo Celso C, Fleming HE, Wu JW, Zhao CX, Miake-Lye S, Fujisaki J, et al. Live-animal tracking of individual haematopoietic stem cells in their niche. Nature. 2009;2009(457):92-6.
16. Li S, Ilaria RL, Million RP, Daley GQ, Van Etten RA. The P190, P210, and P230 forms of the BCR/ABL oncogene induce a similar chronic myeloid leukemia-like syndrome in mice but have different lymphoid leukemogenic activity. J Exp Med. 1999; 189(9): 1399-412.

## Claims

1. Fibronectin, or a functional variant thereof, as well as salts, solvates and/or derivatives thereof, for use in the treatment or prevention of cancer.

2. The fibronectin for use according to claim 1, wherein the cancer is leukemia.

3. The fibronectin for use according to claim 2, wherein the leukemia Chronic Myelogenous Leukemia (CML) or Acute Lymphocytic Leukemia (ALL), and preferably is Bcr-Abl1 positive.

4. The fibronectin for use according to any one of claims 1 to 3, wherein the cancer is an imatinib-resistant leukemia, preferably a Bcr-Abl1 mutant positive leukemia.

5. The fibronectin for use according to claim 4, wherein the Bcr-Abl1 mutant is selected from the group consisting of M351T, F359V, H396P, I432T, F486S, M244V, L248V, G250E, Y253H, Y253F, E255K, K263E, L273M, T315I, F317L, and N331S; and preferably is Bcr-Abl1^{T315I}.

6. The fibronectin for use according to any one of claims 1 to 5, wherein the fibronectin or a functional variant thereof, is either a recombinantly produced protein, or a protein purified from a blood sample from a healthy donor.

7. The fibronectin for use according to any one of the preceding claims, wherein the treatment comprises bone marrow transplantation.

8. The fibronectin for use according to any one of the preceding claims, wherein the treatment comprises the concomitant or sequential use of fibronectin and at least one other anti-cancer agent.

9. The fibronectin for use according to any one of the preceding claims, wherein the treatment comprises the use of a combination of fibronectin or a functional variant thereof, as well as salts, solvates and/or derivatives thereof, and at least one other anti-cancer agent.

10. The fibronectin for use according to claim 8 or 9, wherein at least one anti-cancer agent is a kinase inhibitor, preferably a tyrosine kinase such as a Bcr-Abl kinase and/or Src kinase inhibitor, preferably imatinib or nilotinib, or derivatives and/or salts thereof

11. The fibronectin for use according to any one of the preceding claims, wherein the fibronectin, or a functional variant thereof, as well as salts, solvates and/or derivatives thereof, is administered to a patient suffering from leukemia subsequent or concomitantly to a bone marrow transplantation.

12. A combination comprising (a) fibronectin, or a functional variant thereof, as well as salts, solvates and/or derivatives thereof, and (b) Abl1 -inhibitor.

13. The combination according to claim 12, for use in the treatment of leukemia.

14. A pharmaceutical composition comprising fibronectin, or a functional variant there-of, as well as salts, solvates and/or derivatives thereof, and a pharmaceutically acceptable carrier, stabilizer and/or excipient.

15. The pharmaceutical composition according to claim 14, for use according to any of claims 1 to 11.
